# EUROPEAN PATENT SPECIFICATION

(11) **EP 1 856 132 B1**
(45) Date of publication and mention of the grant of the patent: **01.04.2009**
(21) Application number: 05761235.0
(22) Date of filing: 23.06.2005
(51) Int. Cl.: C07D 513/06

(54) **PROCESS FOR PREPARING SUBSTITUTED BENZOTHIAZINOINDOLES**
VERFAHREN ZUR HERSTELLUNG SUBSTITUIERTER BENZOTHIAZINOINDOLE
PROCEDE POUR PREPARER DES BENZOTHIAZINOINDOLES SUBSTITUES

(30) Priority: 08.03.2005 IN CH02252005
(43) Date of publication of application: 21.11.2007
(73) Proprietor: Suven Life Sciences Limited, Hyderabad 500 034 (Andra Pradesh) (IN)
(72) Inventor: RAMAKRISHNA, Venkata Satya Nirogi, Hyderabad 500 034 Andra Pradesh (IN); SHIRSATH, Vikas Shreekrishna, Hyderabad 500 034 Andra Pradesh (IN); KAMBHAMPATI, Rama Sastri, Hyderabad 500 034 Andra Pradesh (IN); DESHPANDE, Amol Dinkar, Hyderabad 500 034 Andra Pradesh (IN); KOTHMIRKAR, Prabhakar, Hyderabad 500 034 Andra Pradesh (IN); JASTI, Venkateswarlu, Hyderabad 500 034 Andra Pradesh (IN)
(74) Representative: Jennings, Nigel Robin
(86) International application number: PCT/IN2005/000214
(87) International publication number: WO 2006/095360

(56) References cited:
- WO-A-20/04000849
- WO-A-20/04055026
- WO-A-20/05005439

## Description

### Field of the Invention

The present invention provides a process for the preparation of substituted benzothiazinoindoles from a substituted 7-bromoindole derivative, formula **(I),** which is cyclized to obtain compound of formula **(I)** using suitable catalyst and solvents.

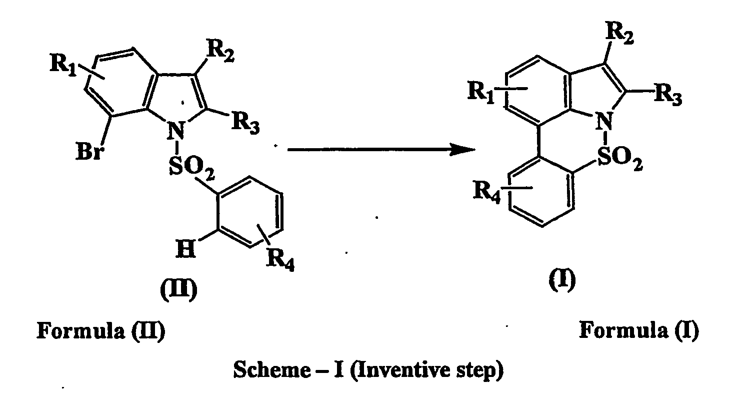

In a preferred embodiment, the invention comprises treating the compound of formula **(II)**, with a suitable palladium (0) or (II) catalyst complex in presence of a suitable base dissolved / suspended in a solvent at suitable temperature range under inert atmosphere / degassed conditions.

### Background of the Invention

The Heck-reaction was first reported by Moritani, Fujiwara and Heck (see references) in the late 1960's. There are several reports on use of intramolecular Heck reaction for the preparation of polycyclic ring systems. Some of these were novel scaffolds were further explored for their potential use in medicine. Our objective was to synthesize compounds having the general structure **(I),** which are useful as medicaments. The initial strategy was as described in Scheme **(II)** below and involved cyclization of substituted 1-(2'-Bromobenzenesulfonyl)indole, using well-known Heck reaction.

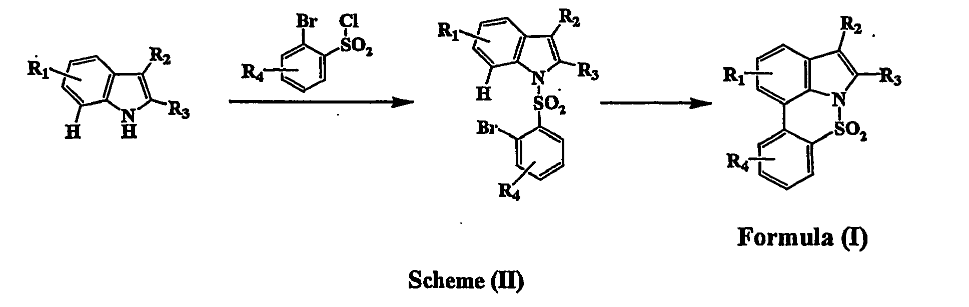

The synthetic approach depicted in Scheme **(II)** above, had several drawbacks;
1. Only a few compounds with the desired tetracyclic ring system could be obtained.
2. The reactant to product conversion rate was low. This issue becomes critical when the final product is being synthesized and the reaction involves catalyst like palladium. This phenomenon was observed particularly when the substitutents on the ring had varying electronic or steric effects.
3. Sometimes the product obtained needed additional efforts to purify.
4. The average yield of all the individual reactions was generally on the lower end.
5. It required large number of various substituted 2-Bromoarylsulfonylchlorides to prepare the compound library. Further, when such multiple substituted aryl sulfonyl chlorides were used, sometimes the reaction did not proceed due to the steric and / or electronic effects of the various substitutions. It should be noted that monosubstituted / disubstituted aryl sulphonyl chlorides are not general utility chemicals and are expensive and synthetically challenging.
6. The special case arises when R₃ is hydrogen; a mixture of two isomeric compounds is possible (see **Scheme IIa** below, R₃ = H); 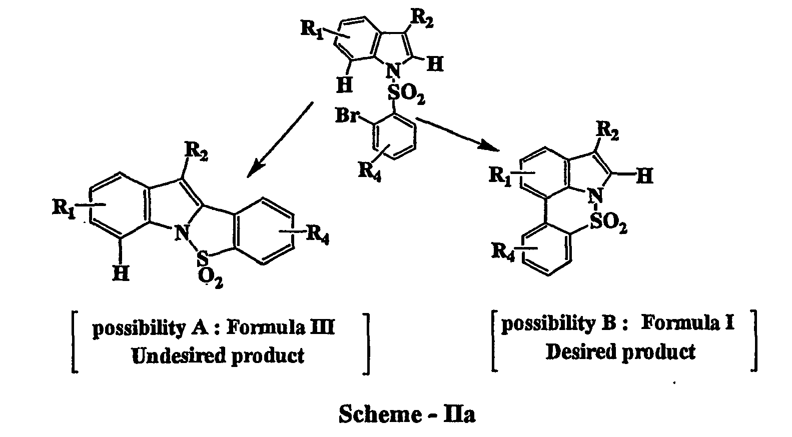
and often the major product is the un-desired tetracyclic ring system, cyclized through C2 of Indole **(Formula III),** which in-turn can be expected due to the favourable steric and thermodynamic factors (Refer WO2004/000849A2, WO2004/055026A1). Further the isolation, purification and even the identification of the two isomeric compounds was a daunting task. Further with the change in substitution pattern in the reactant, the ratio of two isomers obtained would change.

As a result of this, the number of compounds obtained were limited to the availability of particular substituted-2'-Bromoarylsulfonylchlorides. Hence, in order to increase the diversity of substitutions in this ring system we began to explore the new chemistry for the synthesis of ring system defined by compound of formula **(I)**. In those efforts we found that Scheme (I) detailed below, stands to have a better potential in solving the problem of increasing the diversity, purity, yields and economy of synthesis. The key highlight is the use of substituted 7-bromoindoles, which participates in the Heck reaction. The application helps to eliminate numerous limitations stated above and adds in to the diversity of targeted tetracyclic ring system.

The result may be due to the distribution of electrons, ease of formation of complex with palladium catalyst, easier conversion into product due to favourable stereochemistry and other general principles of shifting the equilibrium of the reaction more towards the products thus improving the yields. The reaction favours only one product and the purity of product obtained is good. The starting material, 7-bromoindole once obtained, can be further derivatized suitably. These can be later treated with generally available mono-/di-substituted arylsulfonylchlorides. Thus, the library size and diversity increased significantly fulfilling our main objective.

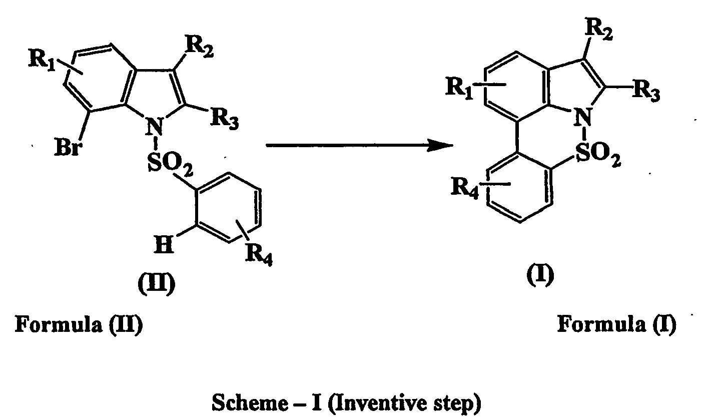

WO 2005/005439 discloses benzothiazino indoles and processes for the preparation thereof.

### Description of the Invention

The present invention provides a process for the preparation of substituted benzothiazinoindoles of general formula **(I),** which comprises of cyclizing the starting material i.e. compound of general formula **(II)** (i.e. substituted 1-benzenesulfonyl-7bromo-1H-indole) suitable catalyst and solvents.

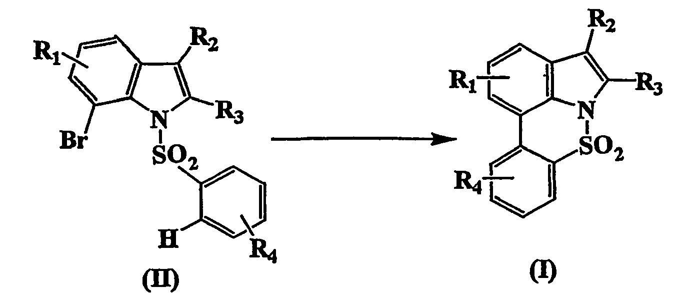

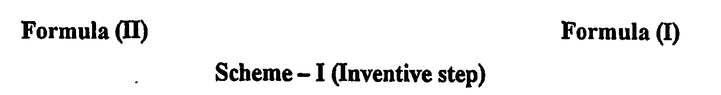

Preferably the suitable catalyst includes a palladium (0) or (II) catalyst complex in presence of appropriate base dissolved/suspended in suitable solvent at suitable temperature range under inert atmosphere/ degassed conditions.

The preferred substitutions for R₁, R₂, R₃ and R₄ for the compounds of formula (I) and (II) are defined as follows: R₁, R₂ and R₄ each independently could be hydrogen, chloro, fluoro, amino, nitro, cyano, CHO, (C₁-C₃)alkyl, perhalo(C₁-C₃)alkyl, (C₁-C₃)alkoxy, aryl, aralkyl, aralkoxy, (C₅-C₇)heterocyclyl, (C₅-C₇)heterocyclylalkyl, (C₅-C₇)heterocyclyloxy, acyl, acetyl, alkylamino, aminoalkyl, amide, hydroxyalkyl, carboxylic acid and its derivatives. R₃ independently could be hydrogen, (C₁-C₃)alkyl, aryl and carboxylic acid and its derivatives.

Suitable catalyst can be any one of those known in the literature which are useful in carrying out ring annulation. Preferred catalyst would include such catalyst which contains Palladium metal in a suitable valency state. More preferable catalyst would be palladium in either (0) or (II) valency state in the form of catalyst complex which may have a ligand/base. It is known that the intramolecular cyclization is carried out by the Pd (0). Thus, the suitable catalyst system preferably can include a Pd compound coupled with a carrier and a base (in some cases), which may give an activated Pd (0) *in-situ* which is stable enough for the reaction to proceed in forward direction. Some examples of such Pd complexes described in literature, are listed below: .
1. Pd (In compounds along with ligand such as phosphines, phosphites, heterocyclic carbene ligands, Li as reported in reference [6]. Pd (II) compounds such as Pd(OAc)₂, PdCl₂, Pd(0)(PPh₃)₄, PdCl₂(PPh₃)₂, Pd₂(dba)₃-CHCl₃, (η³-allyl-PdCl)₂ or Pd on carbon without phosphine ligand. Pd(OAc)₂ and Pd(Cl)₂ are used as precursors of Pd(0) catalysts with or without addition of reducing agents such as phosphine ligands. For example, tetrakis(triphenylphosphine)palladium, Bis(triphenylphosphine)palladium(II) dichloride and (Bis-tri-o-tolylphosphine) palladium. The reference [6] is incorporated herein.
2. Colloidal Pd particles protected with tetraalkylammonium salts,
3. polymer supported active Pd catalyst and
4. Pd on carbon without phosphine.

Both types of Pd compounds namely Pd(0) complexes and Pd (II) salts can be used. It is well established in the literature that catalytic activities of Pd(0) generated in-situ from these Pd compounds are not always the same, and it is advisable to test all of them in order to achieve efficient catalytic reactions.

The ratios of Pd catalyst to ligands are important. It is known that the presence of an excess of ligand leads to decrease in the concentration of active catalyst species thereby inhibiting the catalytic process. Some Pd-catalyzed reactions proceed without phosphine ligands, and a phosphine free catalyst is an ideal one, because phosphines are expensive, difficult to recover and coordinated phosphines as such do not directly participate in the catalytic reaction. Pd being an expensive metal, optimization of its use via recycling is essential.

A suitable base selected should atleast be able to activate the Pd catalyst complex and may serve some secondary role. Such suitable bases includes CH₃COOK, TEA and the like. Preferably these bases should be used in 1 - 5 mole equivalents based on the compound of formula (II), reaction solvent and reaction temperature.

Suitable solvents for the reaction should preferably be inert to reaction conditions, nontoxic and have a high-boiling point. Some examples of suitable solvents are polar aprotic solvents exemplified by dimethyl formamide (DMF), dimethyl sulfoxide (DMSO), dimethylacetamide (DMA) and the like. Generally the solvent having boiling point above 80°C are preferred; examples include dimethylacetamide, dimethylformamide and the like. The amount of suitable used is about 5.- 20 volume/volume.

The inert atmosphere may be maintained by using inert gases such as N₂, Ar or He. Further if needed reaction mixture may be degassed.

The reaction temperature may range from 0 °C to 200 °C based on the choice of solvent and preferably at a temperature of 80 °C 140 °C.

**Summary of the best preferred reaction conditions is as follows:**

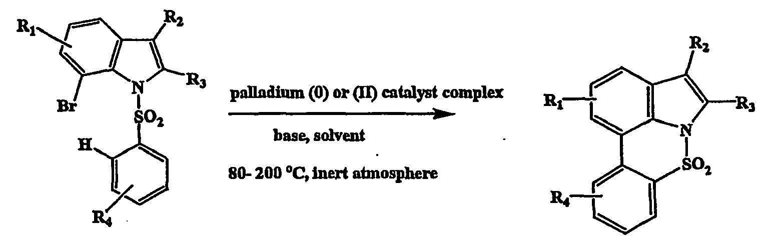

The following description illustrates the method of preparation of variously substituted compounds of general formula **(I)** These are provided by the way of illustration only and therefore should not be construed to limit the scope of the invention.

Commercial reagents were utilized without further purification. Room temperature refers to 25 - 30 °C. Melting points are uncorrected. IR spectra were taken using KBr in solid state and the absorption is expressed in cm⁻¹. Unless otherwise stated, all mass spectra were carried out using ESI conditions. ¹H NMR spectra were recorded at 400 MHz on a Bruker instrument. Deuterated chloroform (99.8 % D) was used as solvent. TMS was used as internal reference standard. Chemical shift values reported herein are expressed in parts per million (δ ppm) values. The following abbreviations are used for the multiplicity for the NMR signals: s=singlet, bs=broad singlet, d=doublet, t=triplet, q=quartet, qui=quintet, h=heptet, dd=doublet doublet, dt=doublet triplet, tt=triplet of triplets, m=multiplet. NMR, mass were corrected for background peaks. Chromatography refers to column chromatography performed using 60 - 120 mesh silica gel and executed under nitrogen pressure (flash chromatography) conditions.

### Example 1: 8-Methyl-3-(4-methyl-piperazin-1-ylmethyl)-1,2-benzothiazino [2,3,4-ab]indole-S,S-dioxide

A mixture of 1-(4-Methylbenzenesulfonyl)-3-(4-methylpiperazin-1-yl-methyl)-7-bromoindole (170 mg, 0.368 m moles), Tetrakis triphenylphosphine palladium (0) (26.0 mg, 0.022 m moles), and potassium acetate (54.0 mg, 0.55 m moles) in dimethyl acetamide (4.0 mL) was heated to 90 - 100 °C under nitrogen atmosphere. The reaction was monitored by TLC for completion. After completion of the reaction (3 - 4 hrs, TLC), the reaction mixture was cooled to 25 °C, and filtered over Hyflow. The filtrate was diluted with ice-water mixture with vigorous stirring. The product was extracted in ethyl acetate (2 x 10 mL) after basification with aqueous KOH to pH, 9 - 10. The combined ethyl acetate extracts were washed with brine, water and dried over magnesium sulfate, the organic volatiles were removed by distillation under reduced pressure, and the crude residue was purified by column chromatography (Silica gel, Ethyl acetate: Triethylamine = 99.5:0.5). The pure compound was obtained as slight brown oil (60 mg, 46 %), which got converted to solids on standing. IR spectra (cm-1) : 799, 1145, 1322,1455; Mass (m/z) : 382 (M+H)+; 1H-NMR : 2.30 (3H, s), 2.39 - 2.58 (8H, m), 2.60 (3H, s), 3.75 (2H, s), 7.42 - 7.48 (2H, m), 7.64 (1H, s), 7.88 - 7.90 (1H, dd), 7.95 - 7.98 (2H, m), 8.10 - 8.12 (1H, d).

### Example 2 : 8-Fluoro-3-(4-methyl-piperazin-1-ylmethyl)-1,2-benzothiazino[2,3,4-ab]indole-S,S-dioxide

A mixture of 1-(4-Fluorobenzenesulfonyl)-3-(4-methylpiperazin-1-yl-methyl)-7-bromoindole (171 mg, 0.368 m moles), Tetrakis triphenylphosphine palladium (0) (26.0 mg, 0.022 m moles), and potassium acetate (54.0 mg, 0.55 m moles) in dimethyl acetamide (4.0 mL) was heated to 130 - 140 oC under nitrogen atmosphere. The reaction was monitored by TLC for completion. This compound was isolated, purified and characterised according to the method described in principle for example 1 above. IR spectra (cm-1) : 801, 1102, 1262, 1323; Mass (m/z) : 386 (M+H)+ ; 1H-NMR : 2.30 (3H, s), 2.37 - 2.60 (8H, m), 3.57 (2H, s), 7.30 - 7.32 (1H, m), 7.47 - 7.51 (1H, t), 7.64 (1H, s), 7.82 - 7.85 (1H, dd), 7.89 - 7.91 (1H, d), 7.94 - 7.96 (1H, d), 8.22 - 8.26 (1H, dd).

### Example 3 : 3-(4-Methylpiperazin-1-ylmethyl)-1,2-benzothiazino[2,3,4-ab]indole-S,S-dioxide

A mixture of 1-(Benzenesulfonyl)-3-(4-methylpiperazin-1-yl-methyl)-7-bromoindole (165 mg, 0.368 m moles), Bis(triphenylphosphine)palladium(II)chloride (8.0 mg, 0.11 m moles), and potassium acetate (54.0 mg, 0.55 m moles) in dimethyl acetamide (4.0 mL) was heated to 110 - 120 oC under nitrogen atmosphere. The reaction was monitored by TLC for completion. This compound was isolated, purified and characterised according to the method described in principle for example 1 above. Mass (m/z) : 368 (M+H)+ .

### Example 4 : 8-Isopropyl-3-(4-methyl-piperazin-1-ylmethyl)-1,2-benzothiazino [2,3,4-ab]indole-S,S-diozide

A mixture of 1-(4-Isopropylbenzenesulfonyl)-3-(4-methylpiperazin-1-yl-methyl)-7-bromoindole (180 mg, 0.368 m moles), Bis(triphenylphosphine)palladium(II)chloride (8.0 mg, 0.011 m moles), and potassium acetate (54.0 mg, 0.55 m moles) in dimethyl acetamide (4.0 mL) was heated to 110 - 120 °C under nitrogen atmosphere. The reaction was monitored by TLC for completion. This compound was isolated, purified and characterised according to the method described in principle for example 1 above. IR spectra (cm⁻¹) : 804, 1172, 1322, 1454; Mass (m/z) : 410 (M+H)⁺; ¹H-NMR : 1.35 - 1.37 (6H, d), 2.29 (3H, s), 2.31 - 2.60 (8H, m), 3.09 - 3.13 (1H, sep.), 3.75 (2H, s), 7.45 - 7.50 (2H, m), 7.64 (1H, s), 7.88 - 7.90 (1H, dd), 7.98 - 8.01 (2H, m), 8.14 - 8.16 (1H, d).

### Example 5 : 1,2-Benzothiazino[2,3,4-ab]indole-S,S-dioxide

A mixture of 1-(13enzenesulfonyl)-7-bromoindole (124 mg, 0.368 m moles), Tetrakis triphenylphosphine palladium (0) (21.0 mg, 0.018 m moles), and potassium acetate (54.0 mg, 0.55 m moles) in dimethyl formamide (4.0 mL) was heated to 90 - 110 °C under nitrogen atmosphere. The reaction was monitored by TLC for completion. This compound was isolated, purified and characterised according to the method described in principle for example 1 above. Mass (m/z) : 256 (M+H)⁺.

### Example 6 : 1,2-Benzothiazino[2,3,4-ab]indole-S,S-dioxide-3-carboxaldehyde

A mixture of 1-Benzenesulfonyl-3-formyl-7-bromo-indole (134 mg, 0.368 m moles), Tetrakis triphenylphosphine palladium (0) (26.0 mg, 0.022 m moles), and potassium acetate (54.0 mg, 0.55 m moles) in dimethyl acetamide (4.0 mL) was heated to 130 - 140°C under nitrogen atmosphere. The reaction was monitored by TLC for completion. This compound was isolated, purified and characterised according to the method described in principle for example 1 above. Mass (m/z) 284 (M+H)⁺.

### Example 7 : 3-Acetyl-1,2-Benzothiazino[2,3,4-ab]indole-S,S-dioxide

A mixture of 1-Benzenesulfonyl-3-acetyl-7-bromo-indole. (140 mg, 0.368 m moles), Tetrakis triphenylphosphine palladium (0) (26.0 mg, 0.022 m moles), and potassium acetate (54.0 mg, 0.55 m moles) in dimethyl acetamide (4.0 mL) was heated to 130 - 140 °C under nitrogen atmosphere. The reaction was monitored by TLC for completion. This compound was isolated, purified and characterised according to the method described in principle for example 1 above. Mass (m/z) : 298 (M+H)⁺.

### Example 8 : 3-(N,N-Dimethylaminoethyl)-1,2-Benzothiazino[2,3,4-ab]indole-S,S-dioxide :

A mixture of [2-(1-Benzenesulfonyl-7-bromo-1H-indol-3-yl)ethyl]dimethylamine (150 mg, 0.368 m moles), Tetrakis triphenylphosphine palladium (0) (26.0 mg, 0.022 m moles), and potassium acetate (54.0 mg, 0.55 m moles) in dimethyl formamide (4.0 mL) was heated to 120 - 140°C under nitrogen atmosphere. The reaction was monitored by TLC for completion. This compound was isolated, purified and characterised according to the method described in principle for example 1 above. I.R. (KBr, cm⁻¹) : 2982, 1594, 1328, 1173, 1128, 756; Mass (m/z) : 327.2 (M+H)⁺; ¹H-NMR : 2.35 (6H, s), 2.69 - 2.72 (2H, m), 2.97 - 3.01 (2H, m), 7.46 - 7.5 (1H, m), 7.59 - 7.64 (2H, m), 7.71 - 7.73 (1H, d, J = 7.72), 7.779 (1H, bm), 7.95 - 7.97 (1H, d, J = 7.68), 8.17 - 8.19 (1H, d, J = 7.92), 8.22 - 8.24 (1H, dd, J = 8.0, 0.96).

### Example 9 : 8-Methyl-3-(N,N-dimethylaminoethyl)-1,2-Benzothiazino[2,3,4-ab]indole-S,S-dioxide

A mixture of {2-[7-Bromo-1-(4-Methylbenzenesulfonyl)-1H-indol-3-yl]ethyl}dimethylamine (155 mg, 0.368 m moles), Tetrakis triphenylphosphine palladium (0) (13.0 mg, 0.011 m moles), and potassium acetate (54.0 mg, 0.55 m moles) in dimethyl acetamide (4.0 mL) was heated to 110 - 130°C under nitrogen atmosphere. The reaction was monitored by TLC for completion. This compound was isolated, purified and characterised according to the method described in principle for example 1 above. Mass (m/z) : 341.2 (M+H)⁺; ¹H-NMR: 2.36 (6H, s), 2.56 (3H, s), 2.69 - 2.73 (2H, t), 2.97 - 3.018.(2H, t), 7.41 - 7.49 (2H, m), 7.585 (1H, s), 7.7 - 7.72 (d, 1H, J=7.8), 7.94 - 7.96 (d, 1H, J = 7.68), 7.981 (1H, s), 8.1- 8.12 (d, 1H, J = 8.12).

### Example 10 : 8-Isopropyl-3-(N,N-dimethylaminoethyl)-1,2-Benzothiazino[2,3,4-ab]indole-S,S-dioxide

A mixture of [2-(1-(4-Isopropylbenzenesulfonyl)-7-bromo-1H-indol-3-yl)ethyl]dimethylamine (165 mg, 0.368 m moles), Bis(triphenylphosphine)palladium (II)chloride (13.0 mg, 0.018 m moles), and potassium acetate (54.0 mg, 0.55 m moles) in dimethyl acetamide (4.0 mL) was heated to 110 - 130 °C under nitrogen atmosphere. The reaction was monitored by TLC for completion. This compound was isolated, purified and characterised according to the method described in principle for example 1 above. Mass (m/z) : 369.4 (M+H)⁺ ; ¹H-NMR : 1.355 - 1.372 (6H, d, J = 6.96), 2.35 (6H, s), 2.69 - 2.73 (2H, m), 2.97 - 3.01 (2H, m), 3.09 - 3.12 (1H, septet, J = 6.92), 7.459 - 7.498 (2H, m), 7.586 (1H, s), 7.7 - 7.72 (dd, 1H, J = 7.8), 7.98 - 7.99 (1H, d, J = 7.64), 8.00 - 8.01 (1H, d, J = 1.52), 8.13 - 8.16 (1H, d, J = 8.24).

### Example 11 : 8-Chloro-3-(N,N-dimethylaminoethyl)-1,2-Benzothiazino[2,3,4-ab]indole-S,S-dioxide

A mixture of [2-(1-(4-Chlorobenzenesulfonyl)-7-bromo-1H-indol-3-yl)ethyl]dimethylamine (162 mg, 0.368 m moles), Tetrakis triphenylphosphine palladium (0) (26.0 mg, 0.022 m moles), and potassium acetate (54.0 mg, 0.55 m moles) in dimethyl acetamide (4.0 mL) was heated to 140 - 150 °C under nitrogen atmosphere. The reaction was monitored by TLC for completion. This compound was isolated, purified and characterised according to the method described in principle for example 1 above. Mass (m/z) : 361.6 (M+H)⁺; ¹H-NMR : 2.35 (6H, s), 2.68 - 2.72 (2H, t), 2.97 -3.01 (2H, t), 7.48 - 7.6 (2H, m), 7.75 - 7.77 (1H, d, J = 7.8), 8.14 - 8.175 (2H, m), 7.92 - 7.94 (1H, d, J = 7.68).

### Example 12 : 8-Fluoro-3-(N,N-dimethylaminoethyl)-1,2-Benzothiazino[2,3,4-ab]indole-S,S-dioxide

A mixture of [2-(1-(4-Fluorobenzenesulfonyl-7-bromo-1H-indol-3-yl)ethyl]dimethylamine (156 mg, 0.368 m moles), Bis(triphenylphosphine) palladium (II)chloride (16.0 mg, 0.022 m moles), and potassium acetate (54.0 mg, 0.55 m moles) in dimethyl acetamide (4.0 mL) was heated to 140 - 160°C under nitrogen atmosphere. The reaction was monitored by TLC for completion. This compound was isolated, purified and characterised according to the method described in principle for example 1 above. Or. (KBr, cm⁻¹) : 2930, 1602, 1463, 1323, 1173, 633; Mass (m/z) : 345.3 (M+H)⁺; ¹H-NMR : 2.38 (6H, s), 2.6- 2.72 (2H, t), 3.0 -3.04 (2H, t), 7.31 -7.32(1H, m), 7.485 -7.523 (1H, t, J= 7.8), 7.596 (1H, s), 7.76 - 7.78 (d, 1H, J = 7.72), 7.81- 7.84 (1H, dd, J = 9.64, 2.48), 7.89 - 7.91 (1H, d, J = 7.68), 8.226 - 8.261 (1H, dd, J = 8.8, 5.28).

### Example 13: 5-Fluoro-8-methyl-3-(N,N-dimethylaminoethyl)-1,2-Benzothiazino[2,3,4-ab]indole-S,S-dioxide

A mixture of [2-(1-(4-Methyl benzenesulfonyl-5-fluoro-7-bromo-1H-indol-3-yl)ethyl]dimethylamine (162 mg, 0.368 m moles), Tetrakis triphenylphosphine palladium (0) (13.0 mg, 0.011 m moles), and potassium acetate (54.0 mg, 0.55 m moles) in dimethyl acetamide (4.0 mL) was heated to 110 - 130 °C under nitrogen atmosphere. The reaction was monitored by TLC for completion. This compound was isolated, purified and characterised according to the method described in principle for example 1 above. M.P : 215 -218 °C ;I.R. (KBr, cm⁻¹) : 2921, 1602,1473, 1316,1171,1136, 533; Mass (m/z) : 359.2 (M+H)⁺; ¹H-NMR : 2.43 (6H, s), 2.56 (3H, s), 2.76 - 2.8 (2H, m), 2.99 -3.03 (2H, m), 7.41 -7.47(3H, m), 7.62 - 7.72 (2H, m), 7.87 (1H, s), 8.09 - 8.11 (1H, d, J = 7.88)

### Example 14 : 5-Chloro-8-Fluoro-3-(N,N-dimethylaminoethyl)-1,2-Benzothiazino[2,3,4-ab]indole-S,S-dioxide

A mixture of [2-(1-(4-Fluoro benzenesulfonyl-5-chloro-7 bromo-1H-indol-3-yl)ethyl]dimethylamine (169 mg, 0.368 m moles), Tetrakis triphenylphosphine palladium (0) (26.0 mg, 0.022 m moles), and potassium acetate (54.0 mg, 0.55 m moles) in dimethyl sulfoxide (4.0 mL) was heated to 160 - 180°C under nitrogen atmosphere. The reaction was monitored by TLC for completion. This compound was isolated, purified and characterised according to the method described in principle for example 1 above. I.R. (KBr, cm⁻¹) : 2763, 1603, 1330, 1174, 1126, 865, 551; Mass(m/z) : 379.2 (M+H)⁺; ¹H-NMR : 2.34 (6H, s), 2.66 - 2.7 (2H, t), 2.93 - 2.96 (2H, m), 7.34 -7.38 (1H, m), 7.63 (1H, s), 7.723 - 7.727 (1H, d, J = 1.52), 7.77 - 7.8(1H, dd, J = 9.4), 7.863 - 7.866 (1H, d, J =1.28), 8.22 - 8.26 (1H, dd, J = 8.6).

### Example 15 : 3-(N,N-Dimethylaminoethyl)-5-chloro-8-methyl-1,2-benzothiazino-[2,3,4-ab]indole-S,S-dioxide

Using essentially the same procedure as described in example 1, above derivative was prepared. Melting Range: 155 - 160°C ;I.R. (KBr, cm⁻¹) : 2923, 1332, 1166, 1136, 810.67; Mass(m/z) : 375.1 (M+H)⁺ ; ¹H-NMR : 2.46 (3H, s), 2.57 (6H, s), 3.03 (2H, bm), 3.049 (2H, bm), 7.46 - 7.48 (1H, bd, J = 7.72), 7.61 (1H, s), 7.71-7.73 (1H, bm), 7.92-7.94 (2H, bm), 8.1 - 8.12 (1H, d, J = 8.16).

### Example 16 : 3-(N,N-Dimethylaminoethyl)-5,8-difluoro-1,2-benzothiazino-[2,3,4-ab]indole-S,S-dioxide :

Using essentially the same procedure as described in example 1, above derivative was prepared. Melting Range ( °C):150 - 152.5; I.R. (KBr, cm¹) : 2976, 1602, 1474, 1336, 1173, 1134, 860, 662, 539; Mass(m/z) : 363.3 (M+H)⁺; ¹H-NMR : 2.34 (6H, s), 2.66 - 2.7 (2H, m), 2.92 - 2.96 (2H; m), 7.358 (1H, m), 7.43 - 7.45 (1H, dd, J = 8.52), 7.59 - 7.62 (1H, dd, J=9.6), 7.64 (1H, s), 7.72 - 7.75 (1H, dd, J = 9.4), 8.22 - 8.26 (dd, 1H, J = 8.8).

### Example 17 : 3-(N,N-Dimethylaminoethyl)-5-fluoro-1,2-benzothiazino-[2,3,4-ab]indole-S,S-dioxide :

Using essentially the same procedure as described in example 1, above derivative was prepared. Melting Range (°C): 126 -129 ;I.R. (KBr, cm⁻¹) : 2953, 1461, 1333, 1173, 1138, 765, 561; Mass (m/z) : 345.3 (M+H)⁺; ¹H-NMR: 2.35 (6H, s), 2.67 - 2.71 (2H, m), 2.92 -2.96 (2H, m), 7.38 - 7.41 (1H, dd, J = 8.56, 2.08), 7.64 - 7.69 (3H, m), 7.78 - 7.8 (1H, m), 8.09 - 8.11 (1H, d, J = 7.92), 8.22 - 8.25 (1H, dd, J = 7.92, 1.00).

### Example 18 : 3-(N,N-Dimethylaminoethyl)-8-methoxy-1,2-benzothiazino-[2,3,4-ab]indole-S,S-dioxide :

Using essentially the same procedure as described in example 1, above derivative was prepared. Melting Range (°C) : 154 -158; I.R.(KBr, cm¹) : 2933, 1595, 1468, 1318, 1166, 1.132, 858; Mass(m/z) : 357.1 (M+H)⁺; ¹H-NMR : 2.35 (6H, s), 2.68 - 2.72 (2H, m), 2.96 - 3.00 (2H, m), 7.1- 7.13 (1H, m), 7.44 -7.48 (1H, t); 7.58 (1H, s), 7.6 - 7.606 (1H, d, J = 2.44), 7.7 -7.72 (1H, d, J = 7.84), 7.9 - 7.92 (1H, d, J = 7.64), 8.14 - 8.16 (d, 1H, J = 8.92).

### Example 19 : 3-(N,N-Dimethylaminoethyl)-5-fluoro-8-methoxy-1,2-benzothiazino-[2,3,4-ab]indole-S,S-dioxide :

Using essentially the same procedure as described in example 1, above derivative was prepared. Melting Range (°C): 186 -187.5 °C; I.R. (KBr, cm⁻¹): 2946, 1597, 1411, 1327, 1168, 1126, 857, 501; Mass (m/z): 375.3 (M+H)⁺; ¹H-NMR: 2.34 (6H,s), 2.66 - 2.7 (2H, m), 2.91 - 2.95 (2H, m), 7.13-7.16 (1H, dd, J = 8.88, 2.44), 7.38 - 7.4(1H, dd, J = 8.56), 7.5 - 7.506 (1H, d, J = 2.4), 7.6 - 7.63 (2H, m), 8.14 - 8.16 (d, 1H, J = 8.88).

### Example 20 : 3-(N,N-Dimethylaminoethyl)-5-fluoro-8-chloro-1,2-benzothiazino-[2,3,4-ab]indole-S,S-dioxide :

Using essentially the same procedure as described in example 1, above derivative was prepared. Melting Range (°C): 152 -153.4; I.R. (KBr, cm⁻¹): 2948, 1333, 1175, 1132, 894, 627; Mass(m/z) : 379.1 (M+H)⁺; ¹H-NMR : 2.348 (6H, s), 2.66 - 2.7 (2H, m), 2.92 -2.96 (2H, m), 7.42 - 7.45 (1H, dd), 7.616 - 7.659 (3H, m, J = 8.48), 8.05 - 8.06 (d, 1H, J = 1.96), 8.15 - 8.176 (1H, d, J = 8.48).

### Example 21 : 3-(N,N-Dimethylaminoethyl)-5-methyl-8-fluoro-1,2-benzothiazino-[2,3,4-ab]indole-S,S-dioxide :

Using essentially the same procedure as described in example 1, above derivative was prepared. I.R. (KBr, cm¹): 2921, 1327, 1176, 786, 762; Mass (m/z) : 359.4 (M+H)⁺; ¹H-NMR : 2.35 (6H, s), 2.68 - 2.72 (2H, m), 2.96 - 3.00 (2H, m), 7.1- 7.13 (1H, m), 7.44 - 7.48 (1H, t), 7.58 (1H, s), 7.60 - 7.606 (1H, d, J = 2.44), 7.7 - 7.72 (1H, d, J = 7.84), 7.9 - 7.92 (1H, d, J = 7.64), 8.14 - 8.16 (1H, d, J = 8.92).

### Example 22 : 3-(N,N-Dimethylaminoethyl)-5-methyl-1,2-benzothiazino-[2,3,4-ab]indole-S,S-dioxide :

Using essentially the same procedure as described in example 1, above derivative was prepared. I.R. (KBr, cm¹): 2919, 1338, 1175, 1127, 786, 763; Mass (m/z): 341.4 (M+H)⁺; ¹H-NMR : 2.4 (6H, s), 2.6 (3H, s), 2.75 - 2.77 (2H, m), 2.99 (2H, m), 7.52 (1H, s), 7.53 (1H, s), 7.59 - 7.63 (1H, m), 7.75 -7.79 (2H, m), 8.16 - 8.18 (1H, d, J = 7.88), 8.21 - 8.23 (1H, dd, J = 9.08)

### Example 23 : 3-(N,N-Dimethylaminoethyl)-5,8-dimethyl-1,2-benzothiazino-[2,3,4-ab]indole-S,S-dioxide :

Using essentially the same procedure as described in example 1, above derivative was prepared. I.R. (KBr, cm⁻¹): 2918, 1463, 1327, 1174, 1139, 807, 607, 527; Mass (m/z): 355.5 (M+H)⁺; ¹H-NMR : 2.4 (6H, s), 2.56 (3H, s), 2.59 (3H, s), 2.73 - 2.77 (2H, m), 2.98 - 3.02 (2H, m), 7.4 - 7.42 (1H, m), 7.51- 7.52 (2H, m), 7.77 (1H, s), 7.96 (1H, s), 8.09 - 8.11 (d, 1H, J = 8.16).

### Example 24 : 3-(N,N-Dimethylaminoethyl)-5-methyl-8-methoxy-1,2-benzothiazino-[2,3,4-ab]indole-S,S-dioxide :

Using essentially the same procedure as described in example 1, above derivative was prepared. I.R (KBr, cm¹): 2918, 1596, 1323, 1303, 1240, 1169, 811, 533; Mass (m/z): 370.7 (M+H)⁺; ¹H-NMR : 2.41 (6H, s), 2.75 - 2.79 (2H, m), 2.98 - 3.29 (2H, m), 3.99 (3H, s), 7.09 - 7.12 (1H, dd, J = 8.84, 2.44), 7.51 - 7.52 (2H, m), 7.57 - 7.58 (1H, d, J = 2.4), 7.72 (1H, s), 8.12 - 8.15 (1H,d,J= 8.8).

### Example 25 : 3-(N,N-Dimethylaminoethyl)-5,8-dichloro-1,2-benzothiazino-[2,3,4-ab]indole-S,S-diozide :

Using essentially the same procedure as described in example 1, above derivative was prepared. Melting range (°C): 101 - 108; I.R (KBr, cm¹): 2982, 2769, 1329, 1178, 1150, 851, 791, 619, 529; Mass (m/z): 395, 397, 399 (M+H)⁺; ¹H-NMR : 2.35 (6H, s), 2.67 - 2.71 (2H, m), 2.93 - 2.97 (2H, m), 7.61 - 7.64 (2H, m), 7.72 - 7.727 (2H, d, J = 1.64), 7.89 - 7.90 (1H, d, J = 1.52), 8.09 - 8.10 (1H, d, J = 1.56), 8.15 - 8.17 (1H, d, J = 8.56).

### Example 26 : 3-(N,N-Dimethylaminoethyl)-5-fluoro-9,10-dichloro-1,2-benzothiazino-[2,3,4-ab]indole-S,S-dioxide :

Using essentially the same procedure as described in example 1, above derivative was prepared. Melting range (°C): 207 - 210; I.R (KBr, cm¹): 2918, 1454, 1340, 1175, 1163, 838, 581; Mass (m/z): 413.2, 415.2 (M+H)⁺; ¹H-NMR : 2.35 (6H, s), 2.66 - 2.70 (2H, m), 2.92 - 2.96 (2H, m), 7.41 - 7.44 (1H, dd, J = 8.4,2.12), 7.59 - 7.62 (1H, dd, J = 9.98, 2.0), 7.68 (1H, s), 7.83 - 7.85 (1H, d, J = 8.78), 7.97 - 7.99 (1H, d, J = 8.78).

### Example 27 : 3-(N,N-Dimethylaminoethyl)-5,9,10-trichloro-1,2-benzothiazino-[2,3,4-ab]indole-S,S-dioxide :

Using essentially the same procedure as described in example 1, above derivative was prepared. Melting range (°C) : 210.4 - 214.9; I.R (KBr, cm¹): 2946, 1445, 1339, 1163, 838, 594, 568; Mass(m/z) : 429.1, 431.1, 432.9 (M+H)⁺; ¹H-NMR : 2.35 (6H, s), 2.67 - 2.71 (2H, m), 2.93 - 2.97 (2H, m), 7.66 (1H, s), 7.70 - 7.71 (1H, d, J = 1.48), 7.83 - 7.86 (2H, m), 8.01-8.03 (1H, d, J = 8.76).

### Example 28 : 3-(N,N-Dimethylaminoethyl)-5-chloro-1,2-benzothiazino-[2,3,4-ab]indole-S,S-dioxide:

Using essentially the same procedure as described in example 1, above derivative was prepared. Melting range (°C) : 133 - 136.3; I.R (KBr, cm¹) : 2962, 1331, 1169, 1124, 826, 766, 749; Mass(m/z) : 361, 363 (M+H)⁺; ¹H-NMR : 2.35 (6H, s), 2.67 - 2.71 (2H, m), 2.93 - 2.97 (2H, m), 7.62 - 7.69 (3H, m), 7.78 - 7.80 (1H, m), 7.92 - 7.93 (1H, d, J = 1.56), 8.12 - 8.14 (1H, d), 8.22 - 8.24 (1H, dd, J = 8.0, 1.08).

### Example 29 : 3-(N,N-Dimethylaminoethyl)-9,10-dichloro-1,2-benzothiazino-[2,3,4-ab] indole-S,S-dioxide :

Using essentially the same procedure as described in example 1, above derivative was prepared. Melting range (°C) : > 240; I.R (KBr, cm¹) : 2940, 1448, 1329, 1165, 793, 600; Mass(m/z) : 394.9, 397 (M+H)⁺; ¹H-NMR : 2.37 (6H, s), 2.71- 2.75 (2H, m), 3.00 - 3.03 (2H, m), 7.45 - 7.49 (1H, t), 7.74 - 7.76 (1H, d, J = 7.72), 7.81 - 7.83 (1H, d, J = 8.76), 7.89 - 7.91 (1H, d, J = 7.76), 8.08 - 8.10 (1H, d, J = 8.72).

### Example 30 : 3-(N,N-Dimethylaminoethyl)-5-chloro-8-methoxy-1,2-benzothiazino-[2,3,4-ab] indole-S,S-dioxide :

Using essentially the same procedure as described in example 1, above derivative was prepared. Melting range (°C) : 176.6 - 183; I.R (KBr, cm¹): 2980, 1596,1311,1167, 850, 573, 534; Mass(m/z) : 391.1 (M+H)⁺; ¹H-NMR : 2.36 (6H, s), 2.67 - 2.71 (2H, m), 2.93 - 2.97 (2H, m), 4.0 (3H, s), 7.13 - 7.16 (1H, dd, J = 8.84,2.36), 7.52 - 7.53 (1H, d, J = 2.36), 7.61 (1H, s), 7.68 - 7.687 (1H, d, J = 1.56), 7.86 - 7.87 (1H, d, J = 1.56), 8.13 - 8.16 (1H, d, J = 8.84).

### Example 31 : 3-(N,N-Dimethylaminoethyl)-5-methyl-8-isopropyl-1,2-benzothiazino-[2,3,4-ab] indole-S,S-dioxide :

Using essentially the same procedure as described in example 1, above derivative was prepared. Melting range (°C) : 86.5 - 93.5; I.R (KBr, cm¹) : 2957, 1599, 1458, 1322, 1174; Mass(m/z) : 383 (M+H)⁺; ¹H-NMR: 1.35 -1.38 (6H, d), 2.36 (6H, s), 2.61 (3H, s), 2.68 - 2.72 (2H, m), 2.94 - 2.98 (2H, m), 3.09 - 3.12 (1H, sep.), 7.46 - 7.48 (1H, dd, J = 8.24, 1.32), 7.49 (1H, s), 7.53 (1H, s), 7.79 (1H, s), 7.98 - 7.99 (1H, d, J =1.61), 8.12 - 8.14 (1H, d, J = 8.28).

### Example 32 : 3-(N,N-Dimethylaminoethyl)-5-fluoro-8-isopropyl-1,2-benzothiazino-[2,3,4-ab] indole-S,S-dioxide :

Using essentially the same procedure as described in example 1, above derivative was prepared. Melting range (°C) : 117 - 124; I.R (KBr, cm¹) : 2917, 1598, 1344, 1178, 1128, 796, 661; Mass(m/z) : 387 (M+H)⁺; ¹H-NMR : 1.35 - 1.37 (6H, d), 2.36 (6H,s), 2.68 - 2.72(2H, m), 2.93 - 2.97 (2H, m), 3.1 - 3.13(1H, sep), 7.38 - 7.4 (1H, dd, J = 8.56,2.1), 7.51 - 7.53 (1H, dd, J =8.28, 1.56), 7.63 (1H, s), 7.68 - 7.71 (1H, dd, J = 9.9,2.08), 7.914 - 7.918 (1H, d, J = 1.48), 8.14 - 8.16 (1H, d, J = 8.28).

### Example 33 : 3-(N,N-Dimethylaminoethyl)-8,10-difluoro-1,2-benzothiazino-[2,3,4-ab] indole-S,S-dioxide :

Using essentially the same procedure as described in example 1, above derivative was prepared. Melting range (°C) : 155 - -160; I.R (KBr, cm¹) : 2963, 1612, 1331, 1261, 1173, 1111, 855, 797, 512; Mass(m/z) : 363.1 (M+H)⁺; ¹H-NMR: 2.41 (6H,s), 2.74 - 2.78(2H, m), 3.02 - 3.06 (2H, m), 7.04 - 7.1 (1H, m), 7.47 - 7.53 (1H, t), 7.62 (1H, s), 7.67 - 7.71 (1H, m), 7.8 - 7.82 (1H, d, J = 7.76),7.87 - 7.89 (1H, d, J = 7.72).

### Example 34 : 3-(N,N-Dimethylaminoethyl)-5,8,10-trifluoro-1,2-benzothiazino-[2,3,4-ab] indole-S,S-dioxide :

Using essentially the same procedure as described in example 1, above derivative was prepared. Melting range (°C) : 140 -144(dec); I.R (KBr, cm¹) : 2917, 1579, 1462, 1339, 1173, 1094, 857, 799, 529; Mass(m/z) : 380.7 (M+H)⁺; ¹H-NMR : 2.38 (6H,s), 2.7 - 2.74(2H, m), 2.95 - 2.99 (2H, m), 7.08 - 7.14 (1H, m), 7.47 - 7.5 (1H, dd,J=8.4,2.04), 7.58 - 7.61 (2H,dd,J=9.84), 7.67 (1H, s).

### Example 35 : 3-(N,N-Dimethylaminoethyl)-5-methyl-9,10-dichloro-1,2-benzothiazino-[2,3,4-ab] indole-S,S-dioxide :

Using essentially the same procedure as described in example 1, above derivative was prepared. I.R (KBr, cm¹): 2936, 1460, 1333, 1175,1132, 816, 609, 594; Mass(m/z) : 409, 411 (M+H)⁺; ¹H-NMR : 2.37 (6H, s), 2.70 - 2.74 (2H, m), 2.96 - 3.00 (2H, m), 7.53 (1H, s), 7.58 (1H, s), 7.70 (1H, s), 7.79 - 7.81 (1H, d, J = 8.8), 8.06 - 8.08 (1H, d, J = 8.82).

### Example 36 : 3-(N,N-Dimethylaminoethyl)-5-chloro-8,10-difluoro-1,2-benzo thiazino-[2,3,4-ab]indole-S,S-dioxide :

Using essentially the same procedure as described in example 1, above derivative was prepared. Or (KBr, cm¹) : 2917, 1339, 1172, 1123, 757; Mass(m/z) : 397.1, 399 (M+H)⁺; ¹H-NMR: 2.42 (6H, s), 2.74 - 2.78 (2H, m), 2.99 - 3.03 (2H, m), 7.08 - 7.14 (1H, m), 7.63 - 7.66 (2H, m), 7.78 - 7.784 (1H, d, J = 1.32), 7.85 - 7.854 (1H, d, J = 1.32).

### Example 37 : 3-(N,N-Dimethylaminoethyl)-5-methyl-8-chloro-1,2-benzothiazino-[2,3,4-ab]indole-S,S-dioxide :

Using essentially the same procedure as described in example 1, above derivative was prepared. I.R (KBr, cm¹) : 2918, 2762, 1330, 1177, 1096, 790; Mass(m/z) : 375, 376.9 (M+H)⁺ ; ¹H-NMR : 2.37 (6H, s), 2.60 (3H, s), 2.69 - 2.73 (2H, m), 2.96 - 3.00 (2H, m), 7.53 - 7.58 (3H, m), 7.74 (1H, s), 8.12 - 8.15 (2H, m).

### Example 38 : 3-(N,N-Dimethylaminoethyl)-5-chloro-8-isopropyl-1,2-benzothiazino-[2,3,4-ab]indole-S,S-dioxide :

Using essentially the same procedure as described in example 1, above derivative was prepared. I.R (KBr, cm¹) : 2963, 1457, 1406, 1336, 1178, 1126, 827; Mass(m/z) : 403.2, 405.2 (M+H)⁺; ¹H-NMR : 1.382 - 1.399 (6H, d, J = 6.8), 2.37 (6H, s), 2.65 - 2.73 (2H, m), 2.94 - 2.98 (2H, m), 3.12 - 3.15 (1H, sep.), 7.53 - 7.56 (1H, dd, J = 8.28), 7.63 (1H, s), 7.694 - 7.699 (1H, d, J =1.68), 7.96 - 7.967 (2H, m), 8.15 - 8.17 (1H, d, J = 8.24).

### Example 39 : 3-(N,N-Dimethylaminoethyl)-5-methyl-8,10-difluoro-1,2-benzothiazino-[2,3,4-ab]indole-S,S-dioxide :

Using essentially the same procedure as described in example 1, above derivative was prepared. I.R (KBr, cm¹) : 2918, 2849, 1619, 1332, 1170, 853, 802; Mass(m/z) : 377.1 (M+H)⁺; ¹H-NMR: 2.45 (6H, s), 2.60 (3H, s), 2.75 - 2.83 (2H, m), 3.10 - 3.60 (2H, m), 7.02 - 7.08 (1H, m), 7.56 - 7.70 (4H, m).

### Example 40 : 3-(N,N-Dimethylaminoethyl)-7-trifluoromethyl-10-chloro-1,2-benzothiazino-[2,3,4-ab]indole-S,S-dioxide :

Using essentially the same procedure as described in example 1, above derivative was prepared. I.R (KBr, cm¹) : 2917, 1348, 1297, 1170, 1142, 753; Mass(m/z) : 429.1, 431 (M+H)⁺; ¹H-NMR: 2.37 (6H, s), 2.72 - 2.76 (2H, m), 2.99 - 3.04 (2H, m), 7.48 - 7.58 (2H, m), 7.72 - 7.82 (2H, m), 8.20 - 8.60 (2H, m).

### Example 41 : 3-(N,N-Dimethylaminoethyl)-5-fluoro-7-trifluoromethyl-10-chloro-1,2-benzothiazino-[2,3,4-ab]indole-S,S-dioxide :

Using essentially the same procedure as described in example 1, above derivative was prepared. I.R (KBr, cm¹) : 2943, 1435, 1349, 1298, 1170, 868; Mass(m/z) : 447.2, 449.2 (M+H)⁺; ¹H-NMR : 2.36 (6H, s), 2.69 - 2.73 (2H, m), 2.94 - 2.98 (2H, m), 7.46 - 7.49 (1H, dd, J = 8.0, 2.1), 7.54 (1H, s), 7.76 - 7.78 (1H, d, J = 8.72), 7.79 - 7.82 (1H, dd), 8.04 - 8.07 (1H, d, J = 8.60).

### Example 42 : 3-(N,N-Dimethylaminoethyl)-5,10-dichloro-7-trifluoromethyl-1,2-benzothiazino-[2,3,4-ab]indole-S,S-dioxide :

Using essentially the same procedure as described in example 1, above derivative was prepared. I.R (KBr, cm¹) : 2926, 1356, 1297, 1173, 1130, 795; Mass(m/z) : 463, 465 (M+H)⁺; ¹H-NMR: 2.45 (6H, s), 2.79 - 2.83 (2H, m), 3.03 - 3.10 (2H, m), 7.538 - 7.549 (1H, s), 7.76 - 7.78 (1H, d, J = 8.64), 7.80 - 7.804 (1H, d, J =1.4), 8.03 (1H, bs), 8.05 - 8.07 (1H, d, J = 8.60).

### REFERENCES

1. Moritani, I., Fujiwara, Y.; Tetrahedron Lett.; 1967 1119-1122.
2. Fujiwara, Y., Moritani, L, Mastuda, M.; Tetrahedron; 1968, 24, 4819-4824.
3. Heck, R F.; J. Am. Chem. Soc.; 1968, 90, 5518-5526.
4. Heck, R F.; J. Am. Chem. Soc.; 1969, 91, 6707-6714.
5. Braese, S., Gil, C., Knepper, K.; Bioorg. Med. Chem.; 2002, 10:8, 2415 - 2438.
6. Amos, P. C., Whiting, D. A.; J. Chem. Soc. Chem. Commun.; 1987, 510-511.

## Claims

1. A process for the preparation of substituted benzothiazinoindoles of general formula (I), 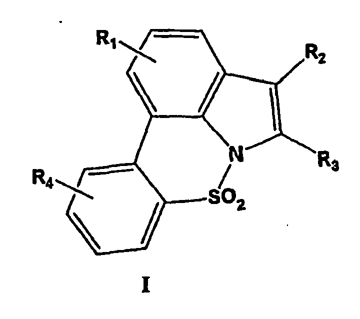 wherein R₁, R₂, and R₄ are independently selected from hydrogen, chloro, fluoro, amino, nitro, Cyano, CHO, (C₁-C₃)alkyl, perhalo(C₁-C₃)alkyl, (C₁-C₃)alkoxy, aryl, aralkyl, aralkoxy, (C₅-C₇)heterocyclyl, (C₅-C₇)heterocyclylalkyl, (C₄-C₇)heterocyclyloxy, acyl, acetyl, alkylamino, aminoalkyl, amide, hydroxyalkyl, carboxylic acid and its derivatives, while R₃ is selected hydrogen, (C₁-C₃)alkyl, aryl and carboxylic acid and its derivatives which comprises of cyclization of compound of general formula-II, (i.e. substituted 1-benzenesulfonyl-7-bromo-1H-indole), 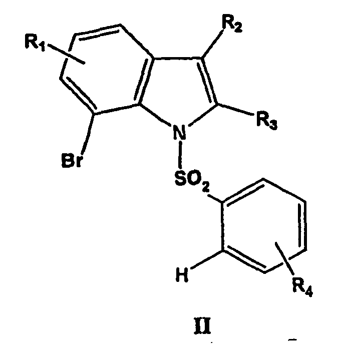 wherein R₁ R₂, R₃ and R₄ are as defined above, using suitable catalyst and solvents at a suitable temperature.

2. A process as claimed in claim-1 wherein said compound of formula I is selected from 8-Methyl-3-(4-methyl-piperazin-1-ylmethyl)-1,2-benzothiazino[2,3,4-ab]indole-S,S-dioxide;
8-Fluoro-3-(4-methyl-piperazin-1-ylmethyl)-1,2-benzothiazino[2,3,4-ab]indole-S,S-dioxide;
3-(4-Methylpiperazin-1-ylmethyl)-1,2-benzothiazino[2,3,4-ab]indole-S,S-dioxide;
8-Isopropyl-3-(4-methyl-piperazin-1-ylmethyl)-1,2-benzothiazino[2,3,4-ab]indole-S,S-dioxide;
1,2-Benzothiazino[2,3,4-ab]indole-S,S-dioxide;
1,2-Benzothiazino[2,3,4-ab]indole-S,S-dioxide;-3-carboxaldehyde;
3-Acetyl-1,2-benzothiazino[2,3,4-ab]indole-S,S-dioxide;
3-(N,N-Dimethylaminoethyl)-1,2-benzothiazino[2,3,4-ab]indole-S,S-dioxide;
8-Methyl-3-(N,N-dimethylaminoethyl)-1,2 benzothiazino[2,3,4-ab]indole-S,S-dioxide;
8-Isopropyl-3-(N,N-dimethylammoethyl)-1,2-benzothiazino[2,3,4-ab]indole-S,S-dioxide;
8-Chloro-3-(N,N-dimethylaminoethyl)-1,2-benzothiazino[2,3,4-ab]indole-S,S-dioxide;
8-Fluoro-3-(N,N-dimethylaminoethyl)-1,2-benzothiazino[2,3,4-ab]indole-S,S-dioxide;
5-Fluoro-8-methyl-3-(N,N-dimethylaminoethyl)-1,2-benzothiazino[2,3,4-ab]indole-S,S-dioxide;
5-Chloro-8-Fluoro-3-(N,N-dimethylaminoethyl)-1,2-benzothiazino[2,3,4-ab]indole-S,S-dioxide;
3-(N,N-Dimethylaminoethyl)-5-chloro-8-methyl-1,2-benzothiazino-[2,3,4-ab]indole-S,S-dioxide;
3-(N,N-Dimethylaminoethyl)-5,8-difluoro-1,2-benzothiazino-[2,3,4-ab]indole-S,S-dioxide;
3-(N,N-Dimethylaminoethyl)-5-fluoro-1,2-benzothiazino-[2,3,4-ab]indole-S,S-dioxide;
3-(N,N-Dimethylaminoethyl)-8-methoxy-1,2-benzothiazino-[2,3,4-ab]indole-S,S-dioxide;
3-(N,N-Dimethylaminoethyl)-5-fluoro-8-methoxy-1,2-benzothiazino-[2,3,4-ab]indole-S,S-dioxide;
3-(N,N-Dimethylaminoethyl)-5-fluoro-8-chloro-1,2-benzothiazino-[2,3,4-ab]indole-S,S-dioxide;
3-(N,N-Dimethylaminoethyl)-5-methyl-8-fluoro-1,2-benzothiazino-[2,3,4-ab]indole-S,S-dioxide;
3-(N,N-Dimethylaminoethyl)-5-methyl-1,2-benzothiazino-[2,3,4-ab]indole-S,S-dioxide;
3-(N,N-Dimethylaminoethyl)-5,8-dimethyl-1,2-benzothiazino-[2,3,4-ab]indole-S,S-dioxide;
3-(N,N-Dimethylaminoethyl)-5-methyl-8-methoxy-1,2-benzothiazino-[2,3,4-ab]indole-S,S-dioxide;
3-(N,N-Dimethylaminoethyl)-5,8-dichloro-1,2-benzothiazino-[2,3,4-ab]indole-S,S-dioxide;
3-(N,N-Dimethylaminoethyl)-5-fluoro-9,10-dichloro-1,2-benzothiazino-[2,3,4-ab]indoleS,S-dioxide;
3-(N,N-Dimethylaminoethyl)-5,9,10-trichloro-1,2-benzothiazino-[2,3,4-ab]indole-S,S-dioxide;
3-(N,N-Dimethylaminoethyl)-5-chloro-1,2 benzothiazino-[2,3,4-ab]indole-S,S-dioxide;
3-(N,N-Dimethylaminoethyl)-9,10-dichloro-1,2-benzothiazino-[2,3,4-ab]indole-S,S-dioxide;
3-(N,N-Dimethylaminoethyl)-5-chloro-8-methoxy-1,2-benzothiazino-[2,3,4-ab]indole-S,S-dioxide;
3-(N,N-Dimethylaminoethyl)-5-methyl-8-isopropyl-1,2-benzothiazino-[2,3,4-ab]indoleS,S-dioxide;
3-(N,N-Dimethylaminoethyl)-5-fluoro-8-isopropyl-1,2 benzothiazino-[2,3,4-ab]indole-S,S-dioxide;
3-(N,N-Dimethylaminoethyl)-8,10-difluoro-1,2-benzothiazino-[2,3,4-ab]indole-S,S-dioxide;
3-(N,N-Dimethylaminoethyl)-5,8,10-trifluoro-1,2-benzothiazino-[2,3,4-ab]indole-S,S-dioxide;
3-(N,N-Dimethylaminoethyl)-5-methyl-9,10-dichloro-1,2 benzothiazino-[2,3,4-ab]indoleS,S-dioxide;
3-(N,N-Dimethylaminoethyl)-5-chloro-8,10-difluoro-1,2-benzothiazino-[2,3,4-ab]indoleS,S-dioxide;
3-(N,N-Dimethylaminoethyl)-5-methyl-8-chloro-1,2-benzothiazino-[2,3,4-ab]indole-S,S-dioxide;
3-(N,N-Dimethylaminoethyl)-5-chloro-8-isopropyl-1,2-benzothiazino-[2,3,4-ab]indoleS,S-dioxide;
3-(N,N-Dimethylaminoethyl)-5-methyl-8,10-fluoro-1,2-benzothiazino-[2,3,4-ab]indoleS,S-dioxide;
3-(N,N-Dimethylaminoethyl)-7-trifluoromethyl-10-chloro-1,2 benzothiazino-[2,3,4-ab]indole-S,S-dioxide;
3-(N,N-Dimethylaminoethyl)-5-fluoro-7-trifluoromethyl-10-chloro-1,2-benzothiazino-[2,3,4-ab]indole-S,S-dioxide;
3-(N,N-Dimethylaminoethyl)-5,10-dichloro-7-trifluoromethyl-1,2-benzothiazino-[2,3,4-ab]indole-S,S-dioxide.

3. A process as claimed in claim 1, wherein the catalyst used for cyclization is a palladium (0) or (II) catalyst complex system.

4. A process as claimed in claim 1, wherein a suitable palladium (0) or (II) catalyst complex system is chosen from one or more of the following : Pd(OAc)₂, PdCl₂, Pd(0)(PPh₃)₄, Pd(0)(P(o-CH₃)Ph₃)₄, PdCl₂(PPh₃)₂, Pd₂(dba)₃-CHCl₃, (η³-allyl-PdCl)₂ or Pd on carbon without phosphine ligand.

5. A process as claimed in any preceding claim, wherein the suitable ligands used with palladium catalyst are selected from one or more of phosphines, phosphites, heterocyclic carbene ligands or Li, and more preferably, phosphines.

6. A process as claimed in any one of claims 3 to 5 wherein the ratio of the preferred Pd catalyst with phosphine catalyst is in the range of 2 to 4 as exemplified in the PdCl₂(PPh₃)₂ and Pd(0)(PPh₃)₄.

7. A process as claimed in any preceding claim, wherein the molar ratio of the palladium catalyst used in the reaction is in the range of 0.01 to 0.10 mole equivalents (1 to 10 mole percent) based on the compound of formula (II), preferably in the range of 0.03 to 0.05 mole equivalents (3 to 5 mole percent) based on the compound of formula (II).

8. A process as claimed in any preceding claim wherein the suitable base is either CH₃COOK, or TEA.

9. A process as claimed in claim 8, wherein the molar ratio of base used in the reaction is 0 - 5 mole equivalents based on the compound of formula (II).

10. A process as claimed in claim 8 or 9, wherein the suitable base is either dissolved or suspended in a polar aprotic solvent such as dimethyl formamide (DMF), dimethyl sulfoxide (DMSO), dimethylacetamide (DMA),) and includes the mixture of these solvents in varying proportions.

11. A process as claimed in any preceding claim wherein the solvent used is dimethylacetamide in the ratio of 5 - 20 volume/volume.

12. A process as claimed in claim 1 wherein one of the solvent used is dimethylformamide in the ratio of from 10 - 100 %, in combination with any other suitable solvent.

13. A process as claimed in claim 1, wherein said cyclization is carried out at a temperature in the range of 0 °C to 200 °C, preferably 120°C to 160°C, based on the solvent

14. A process as claimed in claim 1, wherein said cyclization is carried out under inert atmosphere/ degassed conditions, by using inert gases such as N₂, Ar or He.

## Patentansprüche

1. Verfahren für die Herstellung von substituierten Benzothiazinoindolen der allgemeinen Formel (I), 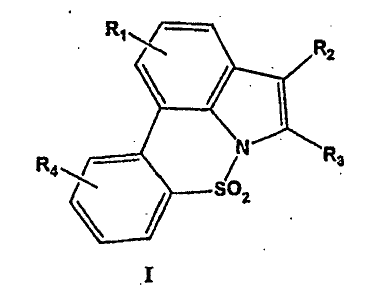 worin R₁, R₂ und R₄ unabhängig ausgewählt sind aus Wasserstoff, Chlor, Fluor, Amino, Nitro, Cyano, CHO, (C₁-C₃) -Alkyl, Perhalogen(C₁-C₃) -alkyl, (C₁-C₃) -Alkoxy, Aryl, Aralkyl, Aralkoxy, (C₅-C-₇)-Heterocyclyl, (C₅-C₇)-Heterocyclylalkyl, (C₅-C₇)-Heterocyclyloxy, Acyl, Acetyl, Alkylamino, Aminoalkyl, Amid, Hydroxyalkyl, Carbonsäure und ihren Derivaten, während R₃ ausgewählt ist aus Wasserstoff, (C₁-C₃)-Alkyl, Aryl und Carbonsäure und ihren Derivaten, welches Cyclisierung von Verbindung der allgemeinen Formel II, (also substituiertem 1-Benzolsulfonyl-7-brom-1H-indol) 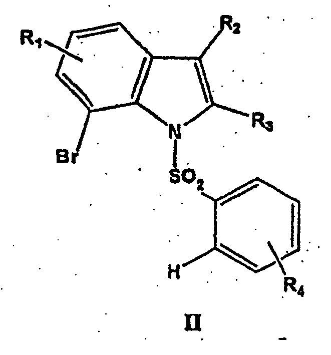 worin R₁, R₂, R₃ und R₄ wie oben definiert sind, unter Verwendung eines geeigneten Katalysators und Lösungsmitteln bei einer geeigneten Temperatur umfasst.

2. Verfahren wie in Anspruch 1 beansprucht, worin besagte Verbindung der Formel I ausgewählt ist aus
8-Methyl-3-(4-methyl-piperazin-1-ylmethyl)-1,2-benzothiazino[2,3,4-ab]indol-S,S-dioxid;
8-Fluor-3-(4-methyl-piperazin-1-ylmethyl)-1,2-benzothiazino[2,3,4-ab]indol-S,S-dioxid;
3-(4-Methylpiperazin-1-ylmethyl)-1,2-benzothiazino[2,3,4-ab]indol-S,S-dioxid;
8-Isopropyl-3-(4-methyl-piperazin-1-ylmethyl)-1,2-benzothiazino[2,3,4-ab]indol-S,S-dioxid;
1,2-Benzothiazino[2,3,4-ab]indol-S,S-dioxid;
1,2-Benzothiazino[2,3,4-ab]indol-S,S-dioxid;-3-carboxaldehyd;
3-Acetyl-1,2-benzothiazino[2,3,4-ab]indol-S,S-dioxid;
3-(N,N-Dimethylaminoethyl)-1,2-benzothiazino[2,3,4-ab]indol-S,S-dioxid
8-Methyl-3-(N,N-dimethylaminoethyl)-1,2-benzothiazino[2,3,4-ab]indol-S,S-dioxid;
8-Isopropyl-3-(N,N-dimethylaminoethyl)-1,2-benzothiazino[2,3,4-ab]indol-S,S-dioxid;
8-Chlor-3-(N,N-dimethylaminoethyl)-1,2-benzothiazino[2,3,4-ab]indol-S,S-dioxid;
8-Fluor-3-(N,N-dimethylaminoethyl)-1,2-benzothiazino[2,3,4-ab]indol-S,S-dioxid;
5-Fluor-8-methyl-3-(N,N-dimethylaminoethyl)-1,2-benzothiazino[2,3,4-ab]indol-S,S-dioxid;
5-Chlor-8-fluor-3-N,N-dimethylaminoethyl)-1,2-benzothiazino[2,3,4-ab]indol-S,S-dioxid;
3-(N,N-Dimethylaminoethyl)-5-chlor-8-methyl-1,2-benzothiazino-[2,3,4-ab]indol-S,S-dioxid;
3-(N,N-Dimethylaminoethyl)-5,8-difluor-1,2-benzothiazino-[2,3,4-ab]indol-S,S-dioxid;
3-(N,N-Dimethylaminoethyl)-5-fluor-1,2-benzothiazino-[2,3,4-ab]indol-S,S-dioxid;
3-(N,N-Dimethylaminoethyl)-8-methoxy-1,2-benzothiazino-[2,3,4-ab]indol-S,S-dioxid;
3-(N,N-Dimethylaminoethyl)-5-fluor-8-methoxy-1,2-benzothiazino-[2,3,4-ab]indol-S,S-dioxid;
3-(N,N-Dimethylaminoethyl)-5-fluor-8-chlor-1,2-benzothiazino-[2,3,4-ab]indol-S,S-dioxid;
3-(N,N-Dimethylaminoethyl)-5-methyl-8-fluor-1,2-benzothiazino-[2,3,4-ab]indol-S,S-dioxid;
3-(N,N-Dimethylaminoethyl)-5-methyl-1,2-benzothiazino-[2,3,4-ab]indol-S,S-dioxid;
3-(N,N-Dimethylaminoethyl)-5,8-dimethyl-1,2-benzothiazino-[2,3,4-ab]indol-S,S-dioxid;
3-(N,N-Dimethylaminoethyl)-5-methyl-8-methoxy-1,2-benzothiazino-[2,3,4-ab]indol-S,S-dioxid;
3-(N,N-Dimethylaminoethyl)-5,8-dichlor-1,2-benzothiazino-[2,3,4-ab]indol-S,S-dioxid;
3-(N,N-Dimethylaminoethyl)-5-fluor-9,10-dichlor-1,2-benzothiazino-[2,3,4-ab]indol-S,S-dioxid;
3-(N,N-Dimethylaminoethyl)-5,9,10-trichlor-1,2-benzothiazino-[2,3,4-ab]indol-S,S-dioxid;
3-(N,N-Dimethylaminoethyl)-5-chlor-1,2-benzothiazino-[2,3,4-ab]indol-S,S-dioxid;
3-(N,N-Dimethylaminoethyl)-9,10-dichlor-1,2-benzothiazino-[2,3,4-ab]indol-S,S-dioxid;
3-(N,N-Dimethylaminoethyl)-5-chlor-8-methoxy-1,2-benzothiazino-[2,3,4-ab]indol-S,S-dioxid;
3-(N,N-Dimethylaminoethyl)-5-methyl-8-isopropyl-1,2-benzothiazino-[2,3,4-ab]indol-S,S-dioxid;
3-(N,N-Dimethylaminoethyl)-5-fluor-8-isopropyl-1,2-benzothiazino-[2,3,4-ab]indol-S,S-dioxid;
3-(N,N-Dimethylaminoethyl)-8,10-difluor-1,2-benzothiazino-[2,3,4-ab]indol-S,S-dioxid;
3-(N,N-Dimethylaminoethyl)-5,8,10-trifluor-1,2-benzothiazino-[2,3,4-ab]indol-S,S-dioxid;
3-(N,N-Dimethylaminoethyl)-5-methyl-9,10-dichlor-1,2-benzothiazino-[2,3,4-ab]indol-S,S-dioxid;
3-(N,N-Dimethylaminoethyl)-5-chlor-8,10-difluor-1,2-benzothiazino-[2,3,4-ab]indol-S,S-dioxid;
3-(N,N-Dimethylaminoethyl)-5-methyl-8-chlor-1,2-benzothiazino-[2,3,4-ab]indol-S,S-dioxid;
3-(N,N-Dimethylaminoethyl)-5-chlor-8₋isopropyl-1,2-benzothiazino-[2,3,4-ab]indol-S,S-dioxid
3-(N,N-Dimethylaminoethyl)-5-methyl-8,10-difluor-1,2-benzothiazino-[2,3,4-ab]indol-S,S-dioxid;
3-(N,N-Dimethylaminoethyl)-7-trifluormethyl-10-chlor-1,2-benzothiazino-[2,3,4-ab]indol-S,S-dioxid;
3-(N,N-Dimethylaminoethyl)-5-fluor-7-trifluormethyl-10-chlor-1,2-benzothiazino-[2,3,4-ab]indol-S,S-dioxid;
3-(N,N-Dimethylaminoethyl)-5,10-dichlor-7-trifluormethyl-1,2-benzothiazino-[2,3,4-ab]indol-S,S-dioxid.

3. Verfahren wie in Anspruch 1 beansprucht, worin der für Cyclisierung verwendete Katalysator ein Palladium (O) oder (II) Katalysatorkomplexsystem ist.

4. Verfahren wie in Anspruch 1 beansprucht, worin ein geeignetes Palladium (O) oder (II) Katalysatorkomplexsystem aus einem oder mehreren der folgenden gewählt wird: Pd(OAc)₂, PdCl₂, Pd(O) (PPh₃)₄, Pd(O) (P(o-CH₃)Ph₃)₄, PdCl₂ (PPh₃)₂, Pd₂ (dba)₃.CHCl₃, (η³-Allyl-PdCl)₂ oder Pd auf Kohlenstoff ohne Phosphinligand.

5. Verfahren wie nach einem vorhergehenden Anspruch beansprucht, worin die geeigneten Liganden, welche mit Palladiumkatalysator verwendet werden, ausgewählt werden aus einem oder mehreren von Phosphinen, Phosphiten, heterocyclischen Carbenliganden oder Li und bevorzugter Phosphinen.

6. Verfahren wie in einem von Ansprüchen 3 bis 5 beansprucht, worin das Verhältnis des bevorzugten Pd-Katalysators mit Phosphinkatalysator im Bereich von 2 bis 4 ist, wie in dem PdCl₂(PPh₃)₂ und Pd(O)(PPh₃)₄ beispielhaft veranschaulicht.

7. Verfahren wie in einem vorhergehenden Anspruch beansprucht, worin das Molverhältnis des in der Umsetzung verwendeten Palladiumkatalysators im Bereich von 0,01 bis 0,10 Moläquivalenten (1 bis 10 Molprozent) basierend auf der Verbindung der Formel (II), vorzugsweise im Bereich von 0,03 bis 0,05 Moläquivalenten (3 bis 5 Molprozent) basierend auf der Verbindung der Formel (II) ist.

8. Verfahren wie in einem vorhergehenden Anspruch beansprucht, worin die geeignete Base entweder CH₃COOK oder TEA ist.

9. Verfahren wie in Anspruch 8 beansprucht, worin das Molverhältnis von in der Umsetzung verwendeten Base 0-5 Moläquivalente basierend auf der Verbindung der Formel (II) ist.

10. Verfahren wie in Anspruch 8 oder 9 beansprucht, worin die geeignete Base entweder in einem polaren aprotischen Lösungsmittel wie Dimethylformamid (DMF), Dimethylsulfoxid (DMSO), Dimethylacetamid (DMA) gelöst oder suspendiert wird und das Gemisch dieser Lösungsmittel in variierenden Anteilen einschließt.

11. Verfahren wie in einem vorhergehenden Anspruch beansprucht, worin das verwendete Lösungsmittel Dimethylacetamid im Verhältnis von 5-20 Volumen/Volumen ist.

12. Verfahren wie in Anspruch 1 beansprucht, worin eines der verwendeten Lösungsmittel Dimethylformamid in einem Verhältnis von 10-100% in Kombination mit einem anderen geeigneten Lösungsmittel ist.

13. Verfahren wie in Anspruch 1 beansprucht, worin besagte Cyclisierung bei einer Temperatur im Bereich von 0°C bis 200°C, vorzugsweise 120°C bis 160°C basierend auf dem Lösungsmittel durchgeführt wird.

14. Verfahren wie in Anspruch 1 beansprucht, worin besagte Cyclisierung unter inerter Atmosphäre/entgasten Bedingungen durch Verwenden von inerten Gasen wie N₂, Ar oder He durchgeführt wird.

## Revendications

1. Procédé de préparation de benzothiazinoindoles de formule générale (I), 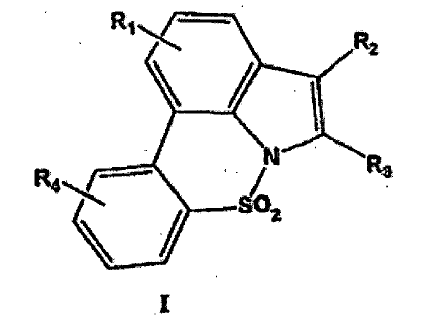 dans laquelle R₁, R₂ et R₄ sont indépendamment choisis parmi un hydrogène, un chloro, un fluoro, un amino, un nitro, un cyano, CHO, un alkyle en C₁ à C₃, un alkyle en C₁ à C₃ perhalogéné, un alcoxy en C₁ à C₃, un aryle, un aralkyle, un aralcoxy, un hétérocyclyle en C₅ à C₇, un hétérocyclylalkyle en C₅ à C₇, un hétérocyclyloxy en C₅ à C₇, un acyle, un acétyle, un alkylamino, un aminoalkyle, un amide, un hydroxyalkyle, un acide carboxylique et ses dérivés, tandis que R₃ est choisi parmi un hydrogène, un alkyle en C₁ à C₃, un aryle et un acide carboxylique et ses dérivés, qui comprend la cyclisation d'un composé de formule générale II, (c'est-à-dire un 1-benzènesulfonyl-7-bromo-1H-indole substitué), 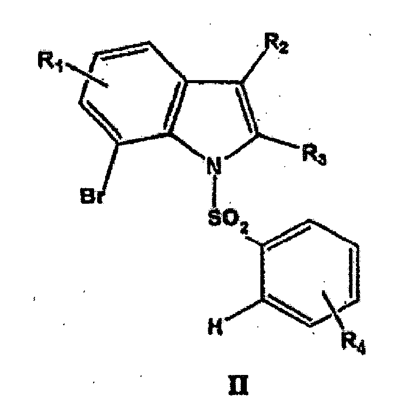 dans laquelle R₁, R₂, R₃ et R₄ sont tels que définis ci-dessus, en utilisant un catalyseur approprié et des solvants appropriés à une température appropriée.

2. Procédé selon la revendication 1, dans lequel ledit composé de formule I est choisi parmi :
le 8-méthyl-3-(4-méthyl-pipérazin-1-ylméthyl)-1,2-benzothiazino[2,3,4-ab]indole-S,S-dioxyde ;
le 8-fluoro-3-(4-méthyl-pipérazin-1-ylméthyl)-1,2-benzothiazino[2,3,4-ab]indole-S,S-dioxyde ;
le 3-(4-méthylpipérazin-1-ylméthyl)-1,2-benzothiazino[2,3,4-ab]indole-S,S-dioxyde ;
le 8-isopropyl-3-(4-méthyl-pipérazin-1-yl-méthyl)-1,2-benzothiazino[2,3,4-ab]indole-S,S-dioxyde ;
le 1,2-benzothiazino[2,3,4-ab]indole-S,S-dioxyde ;
le 1,2-benzothiazino[2,3,4-ab]indole-S,S-dioxyde ;-3-carboxaldéhyde ;
le 3-acétyl-1,2-benzothiazino[2,3,4-ab]indoleS,S-dioxyde ;
le 3-(N,N-diméthylaminoéthyl)-1,2-benzothiazino[2,3,4-ab]indole-S,S-dioxyde ;
le 8-méthyl-3-(N,N-diméthylaminoéthyl)-1,2-benzothiazino[2,3,4-ab]indole-S,S-dioxyde ;
le 8-isopropyl-3-(N,N-diméthylaminoéthyl)-1,2-benzothiazino[2,3,4-ab]indole-S,S-dioxyde ;
le 8-chloro-3-(N,N-diméthylaminoéthyl)-1,2-benzothiazino[2,3,4-ab]indole-S,S-dioxyde ;
le 8-fluoro-3-(N,N-diméthylaminoéthyl)-1,2-benzothiazino[2,3,4-ab]indole-S,S-dioxyde ;
le 5-fluoro-8-méthyl-3-(N,N-diméthylaminoéthyl)-1,2-benzothiazino[2,3,4-ab]indole-S,S-dioxyde ;
le 5-chloro-8-fluoro-3-(N,N-diméthylaminoéthyl)-1,2-benzothiazino[2,3,4-ab]-indole-S,S-dioxyde ;
le 3-(N,N-diméthylaminoéthyl)-5-chloro-8-méthyl-1,2-benzothiazino-[2,3,4-ab]indole-S,S-dioxyde ;
le 3-(N,N-diméthylaminoéthyl)-5,8-difluoro-1,2-benzothiazino-[2,3,4-ab]indole-S,S-dioxyde ;
le 3-(N,N-diméthylaminoéthyl)-5-fluoro-1,2-benzothiazino-[2,3,4-ab]indole-S,S-dioxyde ;
le 3-(N,N-diméthylaminoéthyl)-8-méthoxy-1,2-benzothiazino-[2,3,4-ab]indole-S,S-dioxyde ;
le 3-(N,N-diméthylaminoéthyl)-5-fluoro-8-méthoxy-1,2-benzothiazino-[2,3,4-ab]indole-S,S-dioxyde ;
le 3-(N,N-diméthylaminoéthyl)-5-fluoro-8-chloro-1,2-benzothiazino-[2,3,4-ab]indole-S,S-dioxyde ; 3-(N,N-diméthylaminoéthyl)-5-méthyl-8-fluoro-1,2-benzothiazino-[2,3,4-ab]indole-S,S-dioxyde ;
le 3-(N,N-diméthylaminoéthyl)-5-méthyl-1,2-benzothiazino-[2,3,4-ab]indole-S,S-dioxyde ;
le 3-(N,N-diméthylaminoéthyl)-5,8-diméthyl-1,2-benzothiazino-[2,3,4-ab]indole-S,S-dioxyde ;
le 3-(N,N-diméthylaminoéthyl)-5-méthyl-8-méthoxy-1,2-benzothiazino-[2,3,4-ab]indole-S,S-dioxyde ;
le 3-(N,N-diméthylaminoéthyl)-5,8-dichloro-1,2-benzothiazino-[2,3,4-ab]indole-S,S-dioxyde ;
le 3-(N,N-diméthylaminoéthyl)-5-fluoro-9,10-dichloro-1,2-benzothiazino-[2,3,4-ab]indole-S,S-dioxyde ;
le 3-(N,N-diméthylaminoéthyl)-5,9,10-trichloro-1,2-benzothiazino-[2,3,4-ab]indole-S,S-dioxyde ;
le 3-(N,N-diméthylaminoéthyl)-5-chloro-1,2-benzothiazino-[2,3,4-ab]indole-S,S-dioxyde ;
le 3-(N,N-diméthylaminoéthyl)-9,10-dichloro-1,2-benzothiazino-[2,3,4-ab]indole-S,S-dioxyde ;
le 3-(N,N-diméthylaminoéthyl)-5-chloro-8-méthoxy-1,2-benzothiazino-[2,3,4-ab]indole-S,S-dioxyde ;
le 3-(N,N-diméthylaminoéthyl)-5-méthyl-8-isopropyl-1,2-benzothiazino-[2,3,4-ab]indole-S,S-dioxyde ;
le 3-(N,N-diméthylaminoéthyl)-5-fluoro-8-isopropyl-1,2-benzothiazino-[2,3,4-ab]indole-S,S-dioxyde ;
le 3-(N,N-diméthylaminoéthyl)-8,10-difluoro-1,2-benzothiazino-[2,3,4-ab]indole-S,S-dioxyde ;
le 3-(N,N-diméthylaminoéthyl)-5,8,10-trifluoro-1,2-benzothiazino-[2,3,4-ab]indole-S,S-dioxyde ;
le 3-(N,N-diméthylaminoéthyl)-5-méthyl-9,10-dichloro-1,2-benzothiazino[2,3,4-ab]indole-S,S-dioxyde ;
le 3-(N,N-diméthylaminoéthyl)-5-chloro-8,10-difluoro-1,2-benzothiazino[2,3,4-ab]indole-S,S-dioxyde ;
le 3-(N,N-diméthylaminoéthyl)-5-méthyl-8-chloro-1,2-benzothiazino-[2,3,4-ab]indole-S,S-dioxyde ;
le 3-(N,N-diméthylaminoéthyl)-5-chloro-8-iso-propyl-1,2-benzothiazino-[2,3,4-ab]indole-S,S-dioxyde ;
le 3-(N,N-diméthylaminoéthyl)-5-méthyl-8,10-difluoro-1,2-benzothiazino[2,3,4-ab]indole-S,S-dioxyde ;
le 3-(N,N-diméthylaminoéthyl)-7-trifluorométhyl-10-chloro-1,2-benzothiazino-[2,3,4-ab]indole-S,S-dioxyde ;
le 3-(N,N-diméthylaminoéthyl-5-fluoro-7-trifluorométhyl-10-chloro-1,2-benzothiazino-[2,3,4-ab] indole-S,S-dioxyde ;
le 3-(N,N-diméthylaminoéthyl)-5,10-dichloro-7-trifluorométhyl-1,2-benzothiazino-[2,3,4-ab]indole-S,S-dioxyde.

3. Procédé selon la revendication 1, dans lequel le catalyseur utilisé pour la cyclisation est un système à base d'un complexe catalytique contenant du palladium (0) ou (II).

4. Procédé selon la revendication 1, dans lequel un système à base d'un complexe catalytique contenant du palladium (0) ou (II) approprié est choisi parmi un ou plusieurs des suivants : Pd(OAc)₂, PdCl₂, Pd(0)(PPh₃)₄, Pd(0)(P(o-CH₃)Ph₃)₄, PdCl₂(PPh₃)₂, Pd₂(dba)₃.CHCl₃, (η³-allyl-PdCl)₂ ou du Pd sur charbon sans ligand phosphinique.

5. Procédé selon l'une quelconque des revendications précédentes, dans lequel les ligands appropriés utilisés avec le catalyseur contenant du palladium sont choisis parmi un ou plusieurs des ligands suivants : les phosphines, les phosphites, les ligands à base de carbène hétérocyclique ou Li, et de manière davantage préférée, les phosphines.

6. Procédé selon l'une quelconque des revendications 3 à 5, dans lequel le rapport entre le catalyseur contenant du Pd préféré et le catalyseur phosphinique se situe dans la plage allant de 2 à 4, comme par exemple dans PdCl₂ (PPh₃)₂ et Pf (0) (PPh₃)₄.

7. Procédé selon l'une quelconque des revendications précédentes, dans lequel le rapport molaire du catalyseur contenant du palladium utilisé dans la réaction se situe dans la plage allant de 0,01 à 0,10 équivalent en mole (1 à 10 pour cent en mole) sur la base du composé de formule (II), de préférence dans la plage allant de 0,03 à 0,05 équivalent en mole (3 à 5 pour cent en mole) sur la base du composé de formule (II).

8. Procédé selon l'une quelconque des revendications précédentes, dans lequel la base appropriée est soit CH₃COOK, soit la TEA.

9. Procédé selon la revendication 8, dans lequel le rapport molaire de la base utilisée dans la réaction est de 0 à 5 équivalents en mole sur la base du composé de formule (II).

10. Procédé selon la revendication 8 ou 9, dans lequel la base appropriée est soit dissoute, soit suspendue dans un solvant aprotique polaire, comme le diméthylformamide (DMF), le diméthylsulfoxyde (DMSO), le diméthylacétamide (DMA), y compris dans un mélange de ces solvants en diverses proportions.

11. Procédé selon l'une quelconque des revendications précédentes, dans lequel le solvant utilisé est le diméthylacétamide avec un rapport en volume/volume de 5 à 20.

12. Procédé selon la revendication 1, dans lequel l'un des solvants utilisé est le diméthylformamide avec un rapport de 10% - 100%, en combinaison avec un autre solvant approprié.

13. Procédé selon la revendication 1, dans lequel ladite cyclisation est réalisée à une température située dans la plage allant de 0°C à 200°C, de préférence de 120°C à 160°C, en se basant sur le solvant.

14. Procédé selon la revendication 1, dans lequel ladite cyclisation est réalisée sous atmosphère inerte et dans des conditions dégazées, en utilisant des gaz inertes comme N₂, Ar ou He.
